(19) Europäisches Patentamt
European Patent Office
Office européen des brevets

(11) Publication number: **0 031 561**
**B1**

(12) **EUROPEAN PATENT SPECIFICATION**

(45) Date of publication of patent specification: **10.04.85**

(51) Int. Cl.$^4$: **A 61 K 9/02**

(21) Application number: **80108056.5**

(22) Date of filing: **19.12.80**

(54) **A rectally administered suppository comprising a drug and an adjuvant.**

(30) Priority: **20.12.79 US 105645**

(43) Date of publication of application:
**08.07.81 Bulletin 81/27**

(45) Publication of the grant of the patent:
**10.04.85 Bulletin 85/15**

(84) Designated Contracting States:
**AT BE CH DE FR GB IT LI LU NL SE**

(56) References cited:
**EP-A-0 035 770**
**EP-A-0 036 145**
**EP-A-0 036 534**
**DE-B-1 132 925**
**DE-C- 712 511**
**DE-C- 753 235**
**DE-C- 917 564**
**FR-A-2 293 211**
**FR-A-2 295 754**
**FR-M- 1 650**
**GB-A- 905 092**
**GB-A-1 311 286**
**GB-A-1 483 165**
**GB-A-2 000 025**

(73) Proprietor: **MERCK & CO. INC.**
**126, East Lincoln Avenue P.O. Box 2000**
**Rahway New Jersey 07065 (US)**

(72) Inventor: **Nishihata, Toshiaki**
**University of Kansas 2065 Avenue A**
**Lawrence, Kansas 66044 (US)**
Inventor: **Rytting, Howard J.**
**3009 Topeka Lane**
**Lawrence, Kansas 66044 (US)**
Inventor: **Higuchi, Takeru**
**2811 Schwarz Road**
**Lawrence, Kansas 66044 (US)**

(74) Representative: **Abitz, Walter, Dr.-Ing. et al**
**Abitz, Morf, Gritschneder P.O. Box 86 01 09**
**D-8000 München 86 (DE)**

(56) References cited:
**JOURNAL OF PHARMACEUTICAL SCIENCES, vol. 69, no. 6, June 1980, pages 744-745; T. NISHIHATA et al.: "Enhancement of rectal absorption of drugs by adjuvants"**

**EP 0 031 561 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a rectally administrable suppository for administering drugs to warm blooded animals and particularly to enhancing the rate of absorption of such drugs from the rectal compartment to the blood stream.

One known method of drug administration is accomplished by the incorporation of a drug in a "suppository", which generally speaking, is a medicated solid dosage form generally intended for use in the rectum, vagina, and to a lesser extent, in the urethra. Molded rectal suppositories usually employ vehicles that melt or soften at body temperatures so that the drug may be released for use. On the other hand, soft elastic gelatin capsule suppositories rely on the presence of moisture in the rectum which causes the capsule to open and release its liquid contents which contains its therapeutic agent. Drugs administered in suppository form are administered for either local or systemic effect. The action of the drug is dependent on the nature of the drug, its concentration, and its rate of absorption. Although rectal suppositories are commonly used for the treatment of constipation and hemorrhoids, that is, for local effect, such rectal supppositories are also administered rectally for systemic action. A wide variety of drugs may be rectally administered, as by the use of suppositories, including, for example, analgesics, antispasmodics, sedatives, tranquilizers, and antibacterial agents.

Rectal drug administration has many advantages over other routes of drug administration, such as oral administration and parenteral administration. For example, many drug substances that are given orally undergo inactivation in the stomach because of the acidic, enzymatic content of the stomach or the drug may be subject to digestive attack in the gut and/or to microbial degradation in the lower gut. Oral administration of drugs also directs all of the absorbed substances through the liver where they can be inactivated or reduced in effectiveness.

Rectal administration overcomes wholly, or in part, these known disadvantages of oral drug administration. Rectal drug administration also has advantages over parenteral administration. For example, rectal drug administration does not require highly trained personnel required for parenteral administration and also represents significantly less hazard to the patient.

In view of the known disadvantages of oral and parenteral drug administration, drug administration by rectal delivery enables many drugs to be absorbed from the anorectal area, and yet retain their therapeutic value. The lower hemorrhoidal vein, surrounding the colon and rectum, enters the inferior vena cava and thereby bypasses the liver. Therefore, drugs are absorbed directly into the general circulation when rectally administered. For further background on rectal delivery of drugs, reference is made herein to an article entitled "Rectal Administration of Drugs" by N. Senior, "Advances in Pharmaceutical Sciences", edited by Bean, Beckett, and Corlass, Volume IV, Academic Press (1974) and to Chapter 8, "Suppositories", by Joachim Anschel and Herbert A. Lieberman, Lachman and Lieberman "Theory and Practice of Industrial Pharmacy", Lea and Febiger (1976).

Despite the known advantages of rectal administration of drugs, the rectal administration of drugs is not totally without problems. First, many rectally administered drugs are poorly absorbed while others are slowly absorbed and, if so, are often inactivated or degraded while still in the rectal compartment. It would therefore be highly advantageous if rectally administered drug substances could have their rate of absorption from the rectal compartment to the blood stream enhanced.

FR—1650M describes a solution containing tetracycline, HCl and shicimic acid; GB—A—2,000,025 discloses a solution of nitroimidiazole and mono- or dihydroxybenzoic acid; DE—C—917,564 provides stable solutions of dimethoxymethylfuranchroman with 2,4-dioxybenzoic acid and bases; DE—C—753,235 shows the preparation of aqueous solutions of lactoflavin with 2,4-dioxybenzoic acid and GB—A—1,483,165 teaches the use of sodium salicylate with indomethacin to reduce gastro intestinal effects of the antiinflammatory agent. However, a rectal suppository with enhanced rectal absorbability of the contained drug has not been shown.

## Summary of the invention

It is therefore the object of the present invention to provide an improved rectally administrable suppository which enhances the absorption rate of rectally delivered drugs which can be contained in a soft elastic gelatin capsule or a molded suppository.

Further purposes and objects of this invention will appear as the specification proceeds.

The foregoing objects are accomplished by providing a suppository drug form wherein the absorption rate of rectally administered drugs into the bloodstream of warm blooded animals is enhanced. The rectally administrable suppository comprises a therapeutically effective dosage amount of a drug capable of being absorbed into the bloodstream from the rectal compartment and as an adjuvant hydroxy aryl or hydroxy aralkyl acids of the formula

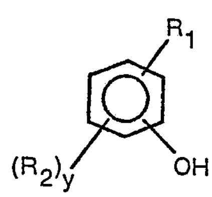

wherein $R_1$ is $CO_2H$, $(CH_2)COOH$,

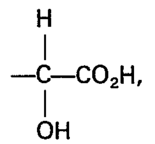

$SO_3H$, or a pharmaceutically acceptable salt thereof wherein $R_2$ is OH, H, alkoxy with 1—10 carbon atoms, alkyl with 1—10 carbon atoms, a halo radical, or a trihalo alkyl with 1—5 carbon atoms, and wherein y is an integer of 1 or 2, with the exception of suppositories containing a combination of indomethacin, niflumic acid, phenylbutazone or acetylsalicylic acid together with salicylic acid or an alkali metal salicylate.

The hydroxy aryl or hydroxy aralkyl acids or pharmaceutically acceptable salts thereof are present in said suppository in an amount sufficient to be effective in enhancing the absorption rate of a drug into the bloodstream from the rectal compartment after rectally administering the drug form to a warm blooded animal.

## Brief description of the drawings

Referring to the accompanying drawings, there is provided four graphs, to be hereinafter described in detail, illustrating the enhanced absorption rate of rectally administered drugs utilizing the present suppository.

Figure 1 is a graph illustrating the enhanced absorption rate of theophylline;

Figure 2 is a graph illustrating the enhanced absorption rate of lidocaine HCl;

Figure 3 is another graph illustrating the enhancement of the absorption rate of lidocaine HCl from the rectal compartment; and

Figure 4 is another graph illustrating enhanced absorption rate of theophylline by rectal administration when utilizing the present suppository.

## Detailed description of the preferred embodiments

The present suppository for enhancing the rate of absorption of drugs from the rectal compartment is useful for a wide range of drugs or drug categories including, but not limited to xanthines, anticancer agents, antibiotics (such as erythromycin, chloramphenicol, penicillin, and cephalosporin), and antiarrythmics (such as guinidine or lidocaine), all of which drugs are capable of being absorbed into the blood stream of a patient from the rectal compartment. Other specific drugs useful in the method and in combination with the hereinafter described adjuvants will be hereinafter identified. The amount of the drug used in the suppository enhancing drug absorption varies over a wide range, generally any therapeutically effective unit dosage amount of the selected drug is used.

The hydroxy aryl or hydroxy aralkyl acids or pharmaceutically acceptable salts thereof, that are used as the adjuvants in our suppositories, have the above-mentioned structural formula including the various isomers possible within the formula. In the formula examples for the alkoxy radical with 1—10 carbon atoms for $R_2$ include methoxy, ethoxy, butoxy, or octyloxy; examples for the alkyl with 1—10 carbon atoms include methyl, isopropyl, ethyl, t-butyl, n-butyl, or t-octyl.

As in the case of the drugs used in our suppositories, the amount of adjuvant used may vary over a wide range; in general, the identity and the amount of the adjuvant used in connection with the drug are selected in order to be effective in enhancing the absorption rate of the drug from the rectal compartment into the bloodstream.

The particular suppositories can have the appropriate size, shape or form of any of the various types of rectal suppositories known to the pharmaceutical art. Useful rectal suppositories include cocoa butter suppositories, synthetic fat suppositories, and gelatin capsules including soft elastic gelatin capsule type suppositories as well as other controlled release devices such as an osmotic pump or other polymeric devices.

A preferred form of suppository comprises a soft elastic gelatin capsule having an outer shell which encloses the drug and the adjuvant in a suitable vehicle which will not attack the walls of the seamless gelatin capsule. The shell encapsulates a preselected drug form and the adjuvant. The gelatin capsule shell may be formulated in accordance with conventional techniques for making filled, seamless, soft elastic gelatin capsules containing therapeutically effective unit dosage amounts of the active drug ingredient. For example, one conventional shell formulation includes about 30—53 parts by weight of gelatin, 15—48 parts by weight of a plasticizer, such as glycerine or sorbitol, and about 16—40 parts by weight of water. Additionally, the gelatin shell may contain preservatives such as mixed parabens, ordinarily methyl or propyl parabens in about a 4:1 ratio. The parabens may be incorporated in the shell formulation in minor proportions as compared to the total weight of the shell formulation. Conventional gelatin capsules utilize gelatin having a bloom value of about 160—200 although this amount may be varied.

In a conventional manner, the gelatin composition is mixed and melted under vacuum conditions. The capsules may be simultaneously formed and filled using a conventional method and apparatus such as disclosed, for example, in U.S. Patent Nos., 1,970,396; 2,288,327; and 2,318,718. The gelatin capsules are

formed into a desired shape and size for insertion into the rectal compartment. It is to be understood, however, that the particular method used for making the soft elastic gelatin shell and for incorporating the fill therein are not considered part of the invention herein.

One of the more important uses of our suppository for rectal administration of drugs is in the administration of sustained release or programmed release drug forms which will slowly release the drug substances into the rectal compartment of a warm blooded animal. The present suppository permits the rapid clearance of the released drug into the blood stream by way of the lower hemorrhoidal vein, instead of moving upwards into the lower gut. This technique thereby reduces or avoids loss of drug effectiveness associated with passage of the drug through the liver.

The following data sets forth specific experiments illustrating various embodiments of the present invention.

Example I

Referring to the accompanying graphs, the effect of salicylic acid on the relative rate of absorption of theophylline from the rectal compartment of a rat is shown in Figure 1. In this experiment, the amount of remaining theophylline, originally at a concentration of about 200 mg/ml, in aqueous solution and continually recirculated or perfused to the rectal compartment of the rat, at the end of one hour, is shown as the lower plot for studies carried out in the presence of 0.2% sodium salicylate, is shown as the upper plot. It is clear that the addition of the salicylate has a marked effect on the rate of absorption of the theophylline into the animal, the adjuvant effect being greatest below a pH of 5.5 and above a pH of 7.5.

A similar accelerating effect on the absorption of the antiarrythmic drug lidocaine by sodium salicylate is evident from Figures 2 and 3 where the amount of the drug absorbed is shown against time and pH. For this drug, the effect of the hydroxyaromatic carboxylate is particularly evident below a pH of 7.0. In fact, below a pH of 6.0, the rate of absorption of the drug by itself approaches zero, whereas in the presence of the salicylate or its acidic form, the absorption rate is substantial and appears to increase at the lower pH values.

With reference to Figure 4, the adjuvant effect on the rectal absorption of these drugs is not limited to salicylic acid alone but is general for the disclosed adjuvants. This adjuvant effect is evident for data obtained for several resorcylic acids and gentisic acid.

Example II

The ability of various of the adjuvants of the present invention to enhance the rate of absorption of significant drugs which are rectally administered, from the rectal compartment into the blood stream, is demonstrated with an *in situ* perfusion method of the rectum. The rectum of a male Sprague-Dowley rat weighing 275—300 g, is exposed by an abdominal incision, a glass cannula is inserted into the distal direction and tied firmly to keep it in position. A second cannula is inserted through the anus 1 cm inside the rectum and secured by ligation. Thus, about 2 cm of the rectum is exposed to the perfusate. The perfustate (6 ml) is circulated at a rate of 2 ml/min at 38°C. As perfustate, 1/15 M—phosphate buffer solutions are used and the ionic strength of the perfusate is adjusted to 0.75 with sodiumchloride as necessary. The adjuvant is employed at a concentration of 0.5%, while the concentration of the various drugs is varied in accordance with the known effective dosages for said drugs. The amount of drug remaining in the perfusate is analyzed as a function of time by appropriate methods, e.g., by high-pressure liquid chromatography. Blood levels are also measured in blood samples taken from a vein in the leg of the rat. The results of this method show that the percent absorption of the various drugs after 1 hour from the perfusate in the rat rectum is significantly enhanced by the presence of an adjuvant of the present invention.

Following is a table of drug and adjuvant combinations which are evaluated in accordance with the method described above:

| Drug | Adjuvant |
|---|---|
| 1. 3,5-diamino-N-(aminoiminomethyl)-6-chloropyrazine-carboxamide (amiloride); | sodium salicylate |
| 2. 6-chloro-3,4-dihydro-2H-1,2,4-benzothiadiazine-7-sulfonamide-1,1-dioxide (hydrochlorothiazide); | sodium homovanillate |
| 3. amiloride hydrochloride and hydrochlorothiazide (moduretic); | 2,5-dihydroxy benzoate |
| 4. S-α-hydrozino-3,4-dihydroxy-α-methylbenzene-propanoic acid monohydrate (carbidopa); | sodium 5-methoxy salicylate |
| 5. carbidopa and 3-hydroxy-L-tyrosine (levodopa) (Sinemet); | sodium 3-methoxy salicylate |

| Drug | Adjuvant |
|---|---|
| 6. (6R-cis)-3-[[(Aminocarbonyloxy]methyl]-7-methoxy-8-oxo-7-[(2-thienylacetyl))amino]-5-thia-1-azabicyclo-[4.2.0]oct-2-ene-2-carboxylic acid (cefoxitin); | sodium salicylate |
| 7. 3-(5H-dibenzo[a,d]cyclohepten-5-ylidene)-N,N-dimethyl-1-propanamine (cyclobenzaprine); | 2,5-dihydroxy benzoate |
| 8. 2′,4′-difluoro-4-hydroxy-[1,1′-biphenyl]-3-carboxylic acid (diflunisal); | sodium homovanillate |
| 9. 1-(p-chlorobenzoyl)-5-methoxy-2-methylindole-3-acetic acid (indomethacin); | sodium homovanillate |
| 10. 3-hydroxy-α-methyl-L-tyrosine (methyldopa); | sodium salicylate |
| 11. (Z)-5-fluoro-2-methyl-1-[[4-(methylsulfinyl)phenyl]methylene]-1H-indene-3-acetic acid (sulindac)′ | sodium 5-methoxy salicylate |
| 12. S-(−)-1-(tert-butylamino)-3-[(4-morpholino-1,2,5-thiadiazol-3-yl)oxy]-2-propanol(timolol); | sodium salicylate |
| 13. (−)-1-(cyclopropylmethyl)-4-[3-(trifluoromethylthio)-5*H*-dibenzo(a,d)cyclohepten-5-ylidene]piperidine hydrochloride | sodium salicylate |
| 14. N-[(S)-1-(ethoxycarbonyl)-3-phenylpropyl]-L-alanyl-L-proline maleate | sodium homovanillate |
| 15. (+)10,11-dihydro-5-methyl-5*H*-dibenzo[a,d]cyclohepten-5,10-imine oxalate | 2,5-dihydroxy benzoate |
| 16. 1-ethyl-6-fluoro-1,4-dihydro-4-oxo-7-(1-piperazinyl)-3-quinolinecarboxylic acid | sodium 5-methoxy salicylate |
| 17. 3-fluoro-*D*-alanine and *D*-4-(1-methyl-3-oxo-1-butenylamino)-3-isoxazolidinone sodium salt hemihydrate | sodium 3-methoxy salicylate |
| 18. *L*-N-(2-oxopiperidin-6-ylcarbonyl)-histidyl-*L*-thiazolidine-4-carboxamide | sodium salicylate |
| 19. N-formimidoyl thienamycin monohydrate | 2,5-dihydroxy benzoate |
| 20. (6,7-dichloro-2-methyl-2-phenyl-1-oxo-5-indanyloxy)acetic acid | sodium homovanillate |
| 21. α-methyl-4-(2-methylpropyl)benzeneacetic acid (ibuprofen) | sodium homovanillate |
| 22. (+)-6-methoxy-α-methyl-2-naphthaleneacetic acid (naproxen) | sodium salicylate |
| 23. 5-(4-chlorobenzoyl)-1.4-dimethyl-1*H*-pyrrole-2-acetic acid | sodium 5-methoxy salicylate |
| 24. 4-butyl-1,2-diphenyl-3,5-pyrazolidinedione (phenylbutazone) | sodium salicylate |
| 25. 9-fluoro-11β,17,21-trihydroxy-16a-methylpregna-1,4-diene-3,20-dione (dexamethasone) | sodium salicylate |
| 26. 11β,17,21-trihydroxypregna-1,4-diene-3,20-dione (prednisolone) | sodium homovanillate |
| 27. 2-(2,6-dichloroanilino)-2-imidazoline (clonidine) | 2,5-dihydroxy benzoate |
| 28. 1-(isopropylamino)-3-(1-naphthyloxy)-2-propanol (propranolol) | sodium 5-methoxy salicylate |
| 29. 7-chloro-1,3-dihydro-1-methyl-5-phenyl-2*H*-1,4-benzodiazepin-2-one (diazepam) | sodium 3-methoxy salicylate |

| Drug | Adjuvant |
|---|---|
| 30. 7-chloro-N-methyl-5-phenyl-3*H*-1,4-benzodiazepin-2-amine 4-oxide (chlorodiazepoxide) | sodium salicylate |
| 31. 5-(aminosulfonyl)-4-chloro-2-[(2-furanylmethyl)amino]benzoic acid (furosemide) | 2,5-dihydroxy benzoate |
| 32. 7-[(hydroxyphenylacetyl)amino]-3-[[(1-methyl-1*H*-tetrazol-5-yl)-thio]methyul]-8-oxo-5-thia-1-azabicyclo[4.2.0]oct-2-ene-2-carboxylic acid (cephamandole) | sodium homovanillate |
| 33. 1-ethyl-1,4-dihydro-7-methyl-4-oxo-1,8-naphthyridine-3-carboxylic acid (nalidixic acid) | sodium homovanillate |
| 34. 6-[[amino(4-hydroxyphenyl)acetyl]-amino]-3,3-dimethyl-7-oxo-4-thia-1-azabicyclo-[3.2.0]heptane-2-carboxylic acid (amoxicillin) | sodium salicylate |
| 35. 4-[4-(4-chlorophenyl)-4-hydroxy-1-piperidinyl]-1-(4-fluorophenyl)-1-butanone (haloperidol) | sodium 5-methoxy salicylate |
| 36. 1-(3-mercapto-2-methyl-1-oxo-propyl)-*L*-proline (captopril) | sodium salicylate |

As already described, the suppository of the present invention for enhancing the rate of absorption of drugs from the rectal compartment is useful for a wide range of drugs. Without limiting the broad applicability of the suppository, there is also pointed out below a number of drugs for which it is particularly useful, thus comprising preferred embodiments of the present invention:

3,5 - diamino - N - (aminoiminomethyl) - 6 - chloropyrazine - carboxamide (amiloride);

6 - chloro - 3,4 - dihydro - 2H - 1,24 - benzothiadiazine - 7 - sulfonamide - 1,1 - dioxide (hydrochlorothiazide);

amiloride hydrochloride and hydrochlorothiazide (Moduretic);

S - α - hydrozino - 3,4 - dihydroxy - α - methylbenzenepropanoic acid monohydrate (carbidopa);

carbidopa and 3 - hydroxy - L - tyrosine (levodopa) (Sinemet);

(6R - cis) - 3 - [[(Aminocarbonyl)oxy]methyl] - 7 - methoxy - 8 - oxo - 7 - [(2 - thienylacetyl)amino] - 5 - thia - 1 - azabicyclo[4.2.0]oct - 2 - ene - 2 - carboxylic acid (cefoxitin);

3 - (5H - dibenzo[a,d]cyclohepten - 5 - ylidene) - N,N - dimethyl - 1 - propanamine (cyclobenzaprine);

2′,4′ - difluoro - 4 - hydroxy[1,1′ - biphenyl] - 3 - carboxylic acid (diflunisal);

1 - (p - chlorobenzoyl) - 5 - methoxy - 2 - methylindole - 3 - acetic acid (indomethacin);

3 - hydroxy - α - methyl - L - tyrosine (methyldopa);

(Z) - 5 - fluoro - 2 - methyl - 1 - [[4 - (methylsulfinyl)phenyl]methylene] - 1H - indene - 3 - acetic aid (sulindac);

S - (−) - 1 - (tert - butylamino) - 3 - [(4 - morpholino - 1,2,5 - thiadiazol - 3 - yl)oxy] - 2 - propanol (timolol);

(−) - 1 - (cyclopropylmethyl) - 4 - [3 - (trifluoromethylthio) - 5*H* - dibenzo(a,d)cyclohepten - 5 - ylidene]piperidine hydrochloride;

N - [(S) - 1(ethoxycarbonyl) - 3 - phenylpropyl] - L - alanyl - L - proline maleate;

(+)10,11 - dihydro - 5 - methyl - 5*H* - dibenzo[a,d]cyclohepten - 5,10 - imine oxalate;

1 - ethyl - 6 - fluoro - 1,4 - dihydro - 4 - oxo - 7 - (1 - piperazinyl) - 3 - quinolinecarboxylic acid;

3 - fluoro - *D* - alaine and *D* - 4 - (1 - methyl - 3 - oxo - 1 - butenylamino) - 3 - isoxazolidinone sodium salt hemihydrate;

*L* - N - (2 - oxopiperidine - 6 - ylcarbonyl) - histidyl - *L* - thiazolidine - 4 - carboxamide;

N - formimidoyl thienamycin monohydrate;

(6,7 - dichloro - 2 - methyl - 2 - phenyl - 1 - oxo - 5 - indanyloxy) acetic acid;

α - methyl - 4 - (2 - methylpropyl)benzeneacetic acid (ibuprofen);

(+) - 6 - methoxy - α - methyl - 2 - naphthaleneacetic acid (naproxen);

5 - (4 - chlorobenzoyl) - 1,4 - dimethyl - 1*H* - pyrrole - 2 - acetic acid;

4 - butyl - 1,2 - diphenyl - 3,5 - pyrazolidinedione (phenylbutazone) not in combination with salicylic acid or an alkali metal salicylate);

9 - fluoro - 11β,17,21 - trihydroxy - 16α - methylpregna - 1,4 - diene - 3,20 - dione (dexamethasone);

11β,17,21,trihydroxypregna - 1,4 - diene - 3,20 - dione (prednisolone);

2 - (2,6 - dichloroanilino) - 2 - imidazoline (clonidine);

1 - (isopropylamino) - 3 - (1 - naphthyloxy) - 2 - propanol (propranolol);

7 - chloro - 1,3 - dihydro - 1 - methyl - 5 - phenyl - 2*H* - 1,4 - benzodiazepin - 2 - one (diazepam);

7 - chloro - N - methyl - 5 - phenyl - 3*H*-1,4 - benzodiazepin - 2 - amine 4 - oxide (chlordiazepoxide);

5 - (aminosulfonyl) - 4 - chloro - 2 - [(2 - furanylmethyl)amino]benzoic acid (furosemide);

7 - [(hydroxyphenylacetyl)amino] - 3 - [[(1 - methyl - 1H - tetrazol - 5 - yl)thio]methyl] - 8 - oxo - 5 - thia - 1 - azabicyclo[4.2.0]oct - 2 - ene - 2 - carboxylic acid (cephamandole);

1 - ethyl - 1,4 - dihydro - 7 - methyl - 4 - oxo - 1,8 - naphthyridine - 3 - carboxylic acid (nalidixic acid)

6 - [[amino(4-hydroxyphenyl)acetyl] - amino] - 3,3 - dimethyl - 7 - oxo - 4 - thia - 1 - azabicyclo[3.2.0]heptane - 2 - carboxylic acid (amoxicillin);

4 - [4 - (4 - chlorophenyl) - 4 - hydroxy - 1 - piperidinyl] - 1 - (4 - fluorophenyl) - 1 - butanone (haloperidol);

1 - (3 - mercapto - 2 - methyl - 1 - oxopropyl) - *L* - proline (captopril)

Example III

A suppository suitable for human use is prepared using the following ingredients:

| Ingredient | Amount |
|---|---|
| A. Sodium 5-methoxy salicylate | 500 mg |
| B. Cefoxitin | 1 g |
| C. Witepsol[R]H-15 suppository excipient[1] [glycerol esters of mixtures of saturated vegetable fatty acids, predominantly lauric acid, derived from purified, specially selected coconut palm kernels, by separation into fatty acid and glyceride portions, fractional distillation of the fatty acid portion, followed by hydrogenation and esterification with glycerin.] | 1.5 g |

[1] Available from Dynamit-Nobel Chemicals, A.G., Troisdorf-Oberlar, Germany.

Dry ingredients A and B are ground together to form a well-mixed, fine powder. Separately, ingredient C is heated to 40—50°C till fluid, after which it is mixed with the fine powder mixture of A and B and poured into a mold to form a unit dosage suppository.

In like manner, the following amounts of various drugs may be incorporated into suppositories using the same excipient and adjuvant and preparation technique described above:

| Drug | Amount |
|---|---|
| hydrochlorothiazide | 75 mg |
| Sinemet (carbidopa and levodopa) | 50/200 mg |
| cyclobenzaprine | 10 mg |
| diflunisal | 250 mg |
| indomethacin | 75 mg |
| methyldopa | 500 mg |
| sulindac | 200 mg |
| ibuprofen | 600 mg |
| naproxen | 250 mg |
| phenylbutazone | 100 mg |
| dexamethasone | 4 mg |
| prednisolone | 25 mg |

| Drug | Amount |
|---|---|
| clonidine | 0.1 mg |
| propranolol | 40 mg |
| diazepam | 5 mg |
| chlorodiazepoxide | 5 mg |
| furosemide | 60 mg |
| cephamandolè | 1000 mg |
| nalidixic acid | 1000 mg |
| amoxicillin | 500 mg |
| haloperidol | 3 mg |
| captopril | 150 mg |
| timolol | 25 mg |
| (−)-1-(cyclopropylmethyl)-4-[3-(trifluoromethylthio)-5*H*-dibenzo(a,d)cyclohepten-5-ylidene]piperidine hydrochloride | 30 mg |
| N-[(S)-1-(ethoxycarbonyl)-3-phenylpropyl]-L-alanyl-L-proline maleate | 25 mg |
| (+)10,11-dihydro-5-methyl-5H-dibenzo[a,d]cyclohepten-5-,10-imine oxalate | 3 mg |
| 1-ethyl-6-fluoro-1,4-dihydro-4-oxo-7-(1-piperazinyl)-3-quinolinecarboxylic acid | 200 mg |
| 3-fluoro-*D*-alanine and *D*-4-(1-methyl-3-oxo-1-butenylamino)-3-isoxazolidinone sodium salt hemihydrate | 500 mg/1500 mg |
| *L*-N-(2-oxopiperidin-6-ylcarbonyl)-histidyl-*L*-thiazolidine-4-carboxamide | 2 mg |
| N-formimidoyl thienamycin monohydrate | 500 mg |
| (6,7-dichloro-2-methyl-2-phenyl-1-oxo-5-indanyloxy) acetic acid | 100 mg |

The adjuvants may be chosen from the following salts or their acids.

Sodium 5-methoxysalicylate
Sodium salicylate
Sodium homovanilate
Sodium 2,5-dihydroxybenzoate
Sodium 2,4-dihydroxybenzoate
Sodium 3,4-dihydroxymandelate
Sodium 3-methoxy-4-hydroxymandelate
Sodium 3-methoxy-4-hydroxycinnamate
Sodium 5-methoxy-2-hydroxyphenylsulfonate
Sodium 3-methylsalicylate
Sodium 5-methylsalicylate
Sodium 5-tert-octylsalicylate
Sodium 3-tert-butyl-6-methylsalicylate
Sodium 3,5-diisopropylsalicylate
Sodium 3-tert-butyl-5-methylsalicylate
Sodium guaicolsulfonate
Sodium 5-bromosalicylate

Sodium 3,5-dibromosalicylate
Sodium 5-iodosalicylate
Sodium 3,5-dibromosalicylate
Sodium 2-hydroxyphenylacetate
Sodium 3-hydroxy-2-naphthoate
Sodium mandelate
Sodium phenyllactate
Sodium 2-hydroxyphenylmethanesulfonate
Sodium 5-trifluoromethyl-2-hydroxybenzoate
Sodium 4-hydroxy-3-hydroxyphenylmethanesulfonate
Sodium 3-methoxysalicylate
Sodium 5-octyloxysalicylate
Sodium 5-butoxysalicylate
Sodium p-hydroxyphenoxyacetate
Sodium 3,4-dihydroxyphenylacetate
Sodium 5-chlorosalicylate
Sodium 3,4-dihydroxycinnamate
Sodium 3,5-dihydroxybenzoate
Sodium 2-hydroxy-3-methoxybenzoate
Sodium 1-hydroxy-2-naphthoate
Sodium salicylurate

Example IV

The ability of various of the adjuvants of the present invention to enhance the rate of absorption of significant drugs which are rectally administered, from the rectal compartment into the blood stream, is demonstrated with an *in situ* perfusion method of the rectum. The rectum of a male Sprague-Dawley rat weighing 275—300 g, is exposed by an abdominal incision, a glass cannula is inserted in the distal direction and tied firmly to keep it in position. A second cannula is inserted through the anus 1 cm. inside the rectum and secured by ligation. Thus, about 2 cm of the rectum is exposed to the perfusate. The perfusate (6 ml) is circulated at a rate of 2 ml/min at 38°C. As perfusate, 1/15 M—phosphate buffer solutions are used and the ionic strength of the perfusate is adjusted to 0.75 with sodium chloride as necessary. The hydroxyaromatic acid adjuvant is employed at a concentration of 0.5%, while the concentration of the various drugs is varied in accordance with the known effective dosages for said drugs. The amount of drug remaining in the perfusate is analyzed as a function of time by appropriate methods, e.g. by high-pressure liquid chromatography. Blood levels are also measured by blood samples taken from a vein in the leg of the rat. The results of this method show that the percent absorption of the various drugs after 1 hour from the perfusate in the rat rectum is significantly enhanced by the presence of the adjuvant of the present invention. Following is a table of drug and adjuvant combinations which are evaluated in accordance with the method described above:

| | |
|---|---|
| 1. 3,7-Dihydro-1,3-dimethyl-1H-purine-2,6-dione (theophylline); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |
| 2. 2-(Diethylamino)-N-(2,6-dimethyl-phenyl) acetamide (lidocaine); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |

| | |
|---|---|
| 3. 3-hydroxy-L-tyrosine (levodopa), (L-Dopa); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |
| 4. Cefmetazole | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate<br>sodium 3,5-dihydroxy benzoate<br>sodium 2,4-dimethoxy benzoate<br>sodium homovanillate<br>sodium 5-methoxy salicylate |

Example V

The ability of various of the hydroxyaromatic acid adjuvants of the present invention to enhance the rate of absorption of significant drugs which are rectally administered, from the rectal compartment into the blood stream, is also demonstrated with an *in vivo* absorption method of the rectum. The rectum of male Sprague-Dawley rat weighing 275—300 g, is exposed by an abdominal incision. A 0.3 ml sample of the drug solution is injected into a 2 cm section of the rectum and the drug solution is maintained in that section by ligating the rectum with thread. Alternatively, 0.3 ml of the drug solution is injected into the rectum using a cannula and the anus is tied firmly to prevent leakage of the drug solution. The hydroxyaromatic acid adjuvant is employed at a concentration of 0.5%, while the concentration of the various drugs is varied in accordance with the known effective dosages for said drugs. The amount of drug present in the blood is analyzed as a function of time by extraction with ether at a pH of less than 2.0 after deproteinization with a 3% trichloroacetic acid solution. Following centrifugation, the ether layer is evaporated and the sediment is dissolved in methanol. This methanol sample containing the drug is assayed by high-pressure liquid chromatography. Blood levels are also measured in blood samples taken from a vein in the leg of the rat using a cannula.

The results of this method show that the percent absorption of the various drugs after one hour in the blood stream is significantly enhanced by the presence of the hydroxyaromatic acid adjuvant of the present invention. Following is a table of drug and adjuvant combinations which are evaluated in accordance with the method described above:

| | |
|---|---|
| 1. 3,7-Dihydro-1,3-dimethyl-1H-purine-2,6-dione (theophylline); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |
| 2. 2-(Diethylamino)-N-(2,6-dimethyl-phenyl) acetamide (lidocaine); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |

| | |
|---|---|
| 3. 3-hydroxy-L-tyrosine (levodopa), (L-Dopa); | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |
| 4. Cefmetazole | Sodium salicylate,<br>sodium benzoate,<br>sodium o-anisate,<br>sodium p-anisate,<br>sodium 3-methoxy salicylate,<br>sodium 2,4-dihydroxy benzoate<br>sodium 2,5-dihydroxy benzoate,<br>sodium 3,5-dihydroxy benzoate<br>sodium 2,4-dimethoxy benzoate<br>sodium homovanillate<br>sodium 5-methoxy salicylate |

Example VI

The ability of various of the adjuvants of the present invention to enhance the rate of absorption of insulin which is rectally administered, from the rectal compartment into the blood stream, is demonstrated with a microenema technique. Insulin is administered to a male Sprague-Dawley rat weighing 275—300 g using a microenema. The microenema is prepared with 0.2 M phosphate buffer, pH 5.0. A volume of 0.3 ml is delivered rectally. Blood samples are taken from a jugular vein of the rat at designated time intervals. The 0.3 ml microenema consists of 5 mg of adjuvant and 1.8 I.U. of insulin. Plasma levels of glucose are measured using the ortho-toluidine method. The results of this method show that plasma glucose levels decrease rapidly after administration of the insulin microenema in the presence of adjuvant. The plasma glucose levels gradually recover from 60 to 120 minutes after administration. The adjuvant greatly enhanced the rectal absorption of insulin. The following is a table of drug and adjuvant combinations which are evaluated in accordance with the method described above:

| Drug | Adjuvant |
|---|---|
| Insuline | Sodium salicylate,<br>sodium 5-methoxy salicylate,<br>sodium 3-methoxy salicylate,<br>sodium homovanillate |

Example VII

The ability of various of the adjuvants of the present invention to enhance the rate of absorption of heparin which is rectally administered, from the rectal compartment into the blood stream, is demonstrated using a microenema technique. A male Sprague-Dawley rat receives 1000 units of heparin (Na salt) dissolved in a 0.1 ml aqueous microenema also containing 20 mg of absorption adjuvant. The results of this method show that there is a dramatic increase in clotting time after rectal administration of heparin with adjuvant. Clotting times were greater than 90 minutes in blood samples taken at 15 minutes through 90 minutes. Following is a table of drug and adjuvant combinations which are evaluated in accordance with the method described above:

| Drug | Adjuvant |
|---|---|
| Heparin | Sodium salicylate,<br>sodium 5-methoxy salicylate,<br>sodium 3-methoxy salicylate,<br>sodium homovanillate |

Example VIII

The ability of various of the adjuvants of the present invention to enhance the rate of absorption of cefmetazole which is rectally administered, from the rectal compartment into the blood stream, is demonstrated using suppository and microenema formulations. Four male beagle dogs weighing 9.5—11 kg are used and are fasted for two days prior to experimentation. Witepsol H-15 is used as an excipient to

prepare the suppository formulation of cefmetazole. Microenema formulations are prepared with aqueous 0.02M phosphate buffer with a final pH of 7.4. Either a 1 g suppository or a 1 ml microenema is given to each dog. The formulations may contain varying amounts of adjuvant. Blood samples are taken from the foreleg vein at predetermined sampling times after dosing. Blood samples are heparinized and centrifuged at 3,000 rpm for 10 minutes. The concentration of cefmetazole in the plasma of the dogs is analyzed as a function of time using plasma aliquots for the assay of cefmetazole. The results of this method show that the inclusion of adjuvant significantly enhanced the rectal absorption of cefmetazole from both microenema and suppository formulations. The suppository formulations provide greater bioavailability of cefmetazole than the microenema formulations as compared with intravenous administration. Following is a table of drugs and adjuvant combinations which are evaluated in accordance with the method described above:

| Drug | Adjuvant |
|---|---|
| Sodium Cefmetazole | Sodium salicylate, sodium 5-methoxy salicylate, sodium homovanillate |

Example IX

The ability of various of the adjuvants of the present invention to enhance the lymphatic transport of water soluble drugs after rectal administration in conjunction with salicylate-based absorption adjuvants is demonstrated with a microenema technique. A male Sprague-Dawley rat weighing 200—225 g is anesthetized with pentobarbital and the thoracic duct is cannulated. Flow through this duct includes the mesenteric drainage. Drugs and absorption adjuvants are delivered in the form of a 0.2 ml aqueous microenema 1/15 M phosphate buffer, at a pH 7.4 (for insulin a pH 5.0). Blood samples (0.3 ml) are taken from the external jugular vein of the rat and centrifuged at 2000 rpm for ten minutes to collect plasma. With the exception of phenol red, determination of drug concentration in plasma and lymph is carried out using a high-pressure liquid chromatography technique at 254 nm. Plasma and lymph samples are deproteinized with acetonitrile. Phenol red is determined at 540 nm after adding 1.0 N sodium hydroxide to the plasma or lymphatic samples. Insulin is assayed with an immunospecific enzyme assay kit supplied by Toyo Jozo Ltd., Japan. The results of this method show that the absorption of the drug in plasma and in lymph collected from the thoracic duct is significantly enhanced by the presence of the hydroxyaromatic acid adjuvant of the present invention. Following is a table of drug and adjuvant combinations which are evaluated in accordance with the method described above:

| Drug | Adjuvant |
|---|---|
| 1. (6R-cis)-3-[[(Aminocarbonyl)oxy]methyl]-7-methoxy-8-oxo-7-[(2-thienylacetyl)amino]-5-thia-1-azabicyclo[4.2.0]oct-2-ene-2-carboxylic acid (cefoxitin); | Sodium 5-methoxy salicylate, sodium salicylate |
| 2. 4,4'-(3H-2,1-Benzoxathiol-3-ylidene)bisphenol S,S-dioxide (phenol red); | Sodium 5-methoxy salicylate, sodium salicylate |
| 3. Insulin | Sodium 5-methoxy salicylate, sodium salicylate |
| 4. 3,7-Dihydro-1,3-dimethyl-1H-purine-2,6-dione (theophylline) | Sodium 5-methoxy salicylate, sodium salicylate |

Example X

The ability of various of the dipeptide and metabolites of epinephrine adjuvants of the present invention to enhance the rate of absorption of significant drugs which are rectally administered, from the rectal compartment into the blood stream, is demonstrated with a microenema technique. A male Sprague-Dawley rat weighing 225—250 g is kept fasting for 16 hours prior to the experiment which is conducted under pentabarbitol anesthesia. During the experiment, the rat is kept on a 38°C surface. The drug with adjuvant is administered rectally as a 0.3 ml microenema using a 0.01 M phosphate buffer at a pH of 7.4 when the depeptide is used as an adjuvant and at a pH of 4.5 when the metabolites of epinephrine are used. Following administration of the drug solution, the rat anus is ligated with thread to avoid leakage of the solution. Blood samples are taken from the jugular vein at regular intervals and centrifuged at 3000 rpm for 10 minutes to provide a plasma sample. The amount of drug absorbed is analyzed as a function of time by appropriate methods, e.g. by high-pressure liquid chromatography. The results of this method show that plasma levels for the various drugs increased significantly after rectal administration in the presence of an adjuvant and relatively high levels were maintained for over 1.5 hours. Following is a table of drug and adjuvant combinations which are evaluated in accordance with the method described above:

| Drug | Adjuvant |
|---|---|
| 1. (6R-cis)-3-[[(Aminocarbonyl)oxy]methyl]-7-methoxy-8-oxo-7-[(2-thienylacetyl)amino]-5-thia-1-azabicyclo [4.2.0]oct-2-ene-2-carboxylic acid (cefoxitin); | Phenylalanyl-phenylalanine, 4-hydroxy-3-methoxymandelic acid, 3,4-dihydroxy-mandelic acid |
| 2. Cefmetazole | phenylalanyl-phenylalanine, 4-hydroxy-3-methoxy-mandelic acid, 3,4-dihydroxy-mandelic acid |
| 3. 4,4'-(3H-2,1-Benzoxathiol-3-ylidene)bisphenol S,S-dioxide (phenol red); | phenylalanyl-phenylalanine, 4-hydroxy-3-methoxy-mandelic acid, 3,4-dihydroxy-mandelic acid |

Example XI

The ability of various of the adjuvants of the present invention to enhance the rate of absorption of significant drugs which are rectally administered, from the rectal compartment into the blood stream, is demonstrated using suppository and microenema formulations. Twelve male beagle dogs weighing 9.5—11 kg are used and are fasted for 36 hours prior to the experiment. The dogs are divided into three groups of four and crossed over with respect to dosage form. Witepsol H-15 is used as an excipient to prepare with 8% gelatin in saline. Suppositories are administered in the amount of 0.5 g. Microenemas are administered with a volume of 0.5 ml. The formulations may contain varying amounts of adjuvant. Blood samples are taken at intervals from the external jugular vein. Blood samples are heparinized and centrifuged at 4000×G for 10 minutes. The plasma levels of the drugs are analyzed as a function of time using plasma aliquots for the assay of drug concentration. The results of this method show that the inclusion of adjuvant significantly enhanced the bioavailability of the drugs in the blood stream. Following is a table of drug and adjuvant combinations which are evaluated in accordance with the method described above:

| Drug | Adjuvant |
|---|---|
| 1. $C_{16}H_{17}KN_2O_4S$ (penicillin G) | Sodium 5-methoxy salicylate, sodium salicylate |
| 2. Gentamycin | Sodium 5-methoxy salicylate, sodium salicylate |
| 3. (6R-cis)-3-[[Aminocarbonyl)oxy]methyl]-7-methoxy-8-amino]-5-thia-1-azabicyclo [4.2.0]oct-2-ene-2-carboxylic acid (cefoxitin) | Sodium 5-methoxy salicylate, sodium salicylate |

Example XII

The ability of various of the hydroxyaromatic acid adjuvants of the present invention to enhance the rate of absorption of insulin which is rectally administered from the rectal compartment into the blood stream, is demonstrated using a microenema technique. A male beagle dog weighing 9.5 to 11 kg is used. Rectal administration of insulin is in the form of a 0.5 ml or a 0.25 ml microenema. Insulin microenema formulation are made up in either 0.9% sodium chloride or in 0.9% sodium chloride with 4% gelatin. Blood samples are taken at intervals for the external jugular vein and treated with EDTA. Blood samples are centrifuged at 4000×G for 10 minutes. Plasma glucose levels are determined at 650 nm using the O-toluidine method. Plasma insulin levels are determined using an immunospecific enzyme assay (Toyo Jozo Company, Ltd., Japan). The results of this method show that the presence of the adjuvant caused a significant decrease in plasma glucose levels concommitant with a large increase in plasma insulin levels. Following is a table of drug and adjuvant combinations which are evaluated in accordance with the method described above:

| Drug | Adjuvant |
|---|---|
| 1. Insulin | Sodium 5-methoxy salicylate, sodium salicylate |

As already described, the methods of the present invention for enhancing the rate of absorption of drugs from the rectal compartment are useful for a wide range of drugs. Without limiting the broad applicability of the novel methods, there are also pointed out a number of drugs for which the novel methods are particularly useful, thus comprising preferred embodiments of the present invention:

3,7 - Dihydro 1,3 - dimethyl - 1H - purine - 2,6 - dione (theophylline);
2 - (Diethylamino) - N - (2,6 - dimethyl - phenyl)acetamide (lidocaine);
3 - hydroxy - L - tyrosine (levodopa) (L-Dopa);
Cefmetazole;
Insuline;
Heparin;
(6R - cis) - 3 - [[(Aminocarbonyl)oxy]methyl] - 7 - methoxy] - 8 - oxo - 7 - [(2 - thienylacetyl)amino] - 5 - thia - 1 - azabicyclo[4.2.0]oct - 2 - ene - 2 - carboxylic acid (cefoxitin);
4,4' - (3H - 2,1 - Benzoxathiol - 3 - ylidene)bisphenol S,S - dioxide (phenol red);
$C_{16}H_{17}KN_2O_4S$ (penicillin G);
Gentamycin.

Example XIII
Preparation of Sodium 2-hydroxy-5-methoxy benzenesulfonate

p-Methoxyphenol (12.4 g) was dissolved in chloroform (100 ml) and cooled in ice. Chlorosulfonic acid (11.6 g) was added dropwise to the stirred reaction mixture. The cooling bath was removed after the addition and stirring continued for 24 hours at room temperature. The chloroform was then evaporated off and the residue was vacuum dried to a hygroscopic light brown solid weighing 20.5 g which was 2-hydroxy-5-methoxy-benzenesulfonic acid.

NMR ($CDCl_3$) and 3.73 (3H, s, $OCH_3$), 6.8—7.2 (3H, m aromatic *H*), and 9.86 (2H, broad s, $OH$ and $SO_3H$). IR (film) 3500—2900, 1512, 1470, 1229, 1198, 996, 938 $cm^{-1}$.

The above sulfonic acid (10 g) was dissolved in water (10 ml) and poured into 75 ml of saturated sodium chloride solution. A white solid separated immediately. It was filtered and dried. Crystallization from water gave the pure sodium salt of 2-hydroxy-5-methoxybenzenesulfonic acid (6.6 g).

NMR ($D_2O$) and 3.83 (3H, s, $OCH_3$), 7.05 and 7.33 (3H, multiplets, aromatic). IR (KBr) 3260, 1518, 1440, 1300, 1280, 1240, 1210, 1905, 1045 $cm^{-1}$.

**Claims for the Contracting States: BE, FR, DE, GB, NL, IT, LU, SE, and CH**

1. A rectally administrable suppository comprising a therapeutically effective dosage amount of a drug capable of being absorbed into the blood stream from a rectal compartment and an adjuvant of the formula:

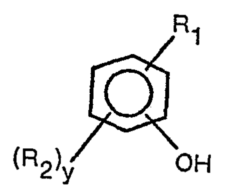

wherein $R_1$ is $CO_2H$, $(CH_2)COOH$,

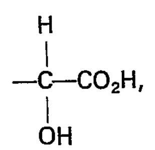

$SO_3H$, or a pharmaceutically acceptable salt thereof wherein $R_2$ is OH, H, alkoxy with 1—10 carbon atoms, alkyl with 1—10 carbon atoms, a halo radical, or a tri- halo alkyl with 1—5 carbon atoms, and wherein y is an integer of 1 or 2, with the exception of rectal suppositories containing a combination of indomethacin, niflumic acid, phenylbutazone or acetylsalicylic acid together with salicylic acid or an alkali metal salicylate.

2. Suppository of Claim 1 wherein said drug comprises xanthines, anticancer agents, antibiotics, polypeptides, antiarrythmics, other cardiovascular agents, antidiabetics, antiulcers, antifungals, antinauseants, sedatives, diuretics, antihypertensives, and drugs for treatment of Parkinson's disease.

3. Suppository of Claim 1 wherein said drug comprises chloramphenicol, penicillin, or cephalosporin.

4. Suppository of Claim 1 wherein said drug comprises guinidine, lidocaine, theophylline, L-dopa, cefmetazole, insulin, cefoxitin, heparin, phenol red, penicillin G, or gentamycin.

5. Suppository of Claim 1 wherein said drug comprises amiloride, hydrochlorothiazide, moduretic, carbidopa, levodopa, cefoxitin, cefmetazole, cyclobenzaprine, diflunisal, indomethacin, methyldopa, sulindac, timolol, (−) - 1 - (cyclopropylmethyl) 4 - [3 - (trifluoromethylthio)5H - dibenzo(a,d)cyclohepten - 5 - ylidene] - piperidine hydrochloride, N - [(S) - 1 - (ethoxycarbonyl) - 3 - phenylpropyl] - L - alanyl - L - proline maleate, (+)10,11 - dihydro - 5 - methyl - 5H - dibenzo[a,d]cyclohepten - 5,10 - imine oxalate, 1 - ethyl - 6 - fluoro - 1,4 - dihydro - 4 - oxo - 7 - (1 - piperazinyl) - 3 - quinoline - carboxylic acid, 3 - fluoro - *D* - alanine and *D* - 4 - (1 - methyl - 3 - oxo - 1 - butenylamino) - 3 - isoxazolidinone sodium salt hemihydrate, *L* - N - (2 - oxopiperidin - 6 -

ylcarbonyl) - histidyl - *L* - thiazolidine - 4 - carboxamide, N - formimidoyl thienamycin monohydrate, (6,7 - dichloro - 2 - methyl - 2 - phenyl - 1 - oxo - 5 - indanyloxy)acetic acid, ibuprofen, naproxen, 5 - (4 - chlorobenzoyl) - 1,4 - dimethyl - 1*H* - pyrrole - 2 - acetic acid, phenylbutazone, dexamethasone, prednisolone, clonidine, propranolol, diazepam, chlorodiazeproxide, furosemide, cephamandole, nalidixic acid, amoxicillin, haloperidol, and captopril.

6. Suppository of any one of Claims 1 to 5 wherein said adjuvant is 5 - methoxysalicylic acid, salicylic acid, homovanillic acid; 2,5 - dihydroxybenzoic acid; 2,4 - dihydroxybenzoic acid; 3,4 - dihydroxymandelic acid; 3 - methoxy - 4 - hydroxy - cinnamic acid; 5 - methoxy - 2 - hydroxyphenylsulfonic acid; 3 - methylsalicylic acid; 5 - methylsalicylic acid; 5 - tert - octylsalicylic acid; 3 - tert - butyl - 6 - methylsalicylic acid; 3,5 - diisopropylsalicylic acid; 3 - tert - butyl - 5 - methylsalicylic acid; guaicolsulfonic acid; 5 - bromosalicylic acid; 3,5 - dibromosalicylic acid; 5 - iodosalicylic acid; 3,5 - diiodosalicylic acid; 2 - hydroxyphenylacetic acid; 3 - hydroxy - 2 - naphthoic acid; mandelic acid; phenyllactic acid; 2 - hydroxyphenylmethanesulfonic acid; 5 - trifluoro - methyl - 2 - hydroxybenzoic acid; 4 - hydroxy - 3 - hydroxyphenylmethanesulfonic acid; 3 - methoxy - salicylic acid; 5 - octyloxysalicylic acid; 5 - butoxy - salicylic acid; p - hydroxyphenoxyacetic acid; 3,4 - dihydroxyphenylacetic acid; 5 - chlorosalicylic acid; 3,4 - dihydroxycinnamic acid; 3,5 - dihydroxybenzoic acid; 2 - hydroxy - 3 - methoxybenzoic acid; 1 - hydroxy - 2 - naphthoic acid; salicyluric acid; or the sodium salts thereof.

7. Suppository of any one of Claims 1 to 6 comprising a soft elastic gelatin capsule containing said drug and said adjuvant.

**Claims for the Contracting State AT:**

1. A process for preparing a rectally administrable suppository by formulating in a manner known per se a therapeutically effective dosage amount of a drug capable of being absorbed into the blood stream from the rectal compartment and an adjuvant of the formula:

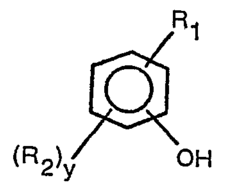

wherein $R_1$ is $CO_2H$, $(CH_2)COOH$,

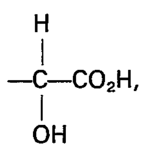

$SO_3H$, or a pharmaceutically acceptable salt thereof wherein $R_2$ is OH, H, alkoxy with 1—10 carbon atoms, lower alkyl with 1—10 carbon atoms, a halo radical, or a tri-halo alkyl with 1—5 carbon atoms, and wherein y is an integer of 1 or 2, with the exception of a combination of indomethacin, niflumic acid, phenylbutazone or acetylsalicylic acid together with salicylic acid or an alkali metal salicylate.

2. The process of Claim 1 wherein said drug comprises xanthines, anticancer agents, antibiotics, polypeptides, antiarrythmics, other cardiovascular agents, antidiabetics, antiulcers, antifungals, antinauseants, sedatives, diuretics, antihypertensives, and drugs for treatment of Parkinson's disease.

3. The process of Claim 1 wherein said drug comprises chloramphenicol, penicillin, or cephalosporin.

4. The process of Claim 1 wherein said drug comprises quinidine, lidocaine, theophylline, L-dopa, cefmetazole, insulin, cefoxitin, heparin, phenol red, penicillin G, or gentamycin.

5. The process of Claim 1 wherein said drug comprises amiloride, hydrochlorothiazide, moduretic, carbidopa, levodopa, cefoxitin, cefmetazole, cyclobenzaprine, diflunisal, indomethacin, methyldopa, sulindac, timolol, (−) - 1 - (cyclopropylmethyl)4 - [3 - (trifluoromethylthio) 5H - dibenzo(a,d) - cyclohepten - 5 - ylidene]piperidine hydrochloride, N - [(S) - 1 - (ethoxycarbonyl) - 3 - phenylpropyl] - L - alanyl - L - proline maleate, (+)10,11 - dihydro - 5 - methyl - 5H - dibenzo[a,d]cyclohepten - 5,10 - imine oxalate, 1 - ethyl - 6 - fluoro - 1,4 - dihydro - 4 - oxo - 7 - (1 - piperazinyl) - 3 - quinoline - carboxylic acid, 3 - fluoro - *D* - alanine and *D* - 4(1 - methyl - 3 - oxo - 1 - butenylamino) - 3 - isoxazolidinone sodium salt hemihydrate, *L* - N - (2 - oxopiperidin - 6 - ylcarbonyl) - histidyl - *L* - thiazolidine - 4 - carboxamide, N - formimidoyl thienamycin monohydrate, (6,7 - dichloro - 2 - methyl - 2 - phenyl - 1 - oxo - 5 - indanyloxy) acetic acid, ibuprofen, naproxen, 5 - (4 - chlorobenzoyl) - 1,4 - dimethyl - 1*H* - pyrrole - 2 - acetic acid, phenylbutazone, dexamethasone, prednisolone, clonidine, propranolol, diazepam, chlorodiazeproxide, furosemide, cephamandole, nalidixic acid, amoxicillin, haloperidol, and captopril.

6. The process of any one of Claims 1 to 5 wherein said adjuvant is 5 - methoxysalicylic acid, salicylic acid, homovanillic acid; 2,5 - dihydroxybenzoic acid; 2,4 - dihydroxybenzoic acid; 3,4 - dihydroxy-

mandelic acid; 3 - methoxy - 4 - hydroxycinnamic acid; 5 - methoxy - 2 - hydroxyphenylsulfonic acid; 3 - methylsalicylic acid; 5 - methylsalicylic acid; 5 - tert - octylsalicylic acid; 3 - tert - butyl - 6 - methylsalicylic acid; 3,5 - diisopropylsalicylic acid; 3 - tert - butyl - 5 - methylsalicylic acid; guaicolsulfonic acid; 5 - bromosalicylic acid; 3,5 - dibromosalicylic acid; 5 - iodosalicylic acid; 3,5 - diiodosalicylic acid; 2 - hydroxyphenylacetic acid; 3 - hydroxy - 2 - naphthoic acid; mandelic acid; phenyllactic acid; 2 - hydroxyphenylmethanesulfonic acid; 5 - trifluoromethyl - 2 - hydroxybenzoic acid; 4 - hydroxy - 3 - hydroxyphenyl - methanesulfonic acid; 3 - methoxysalicylic acid; 5 - octyloxysalicylic acid; 5 - butoxysalicylic acid; p - hydroxyphenoxyacetic acid; 3,4 - dihydroxyphenylacetic acid; 5 - chlorosalicylic acid; 3,4 - dihydroxy - cinnamic acid; 3,5 - dihydroxybenzoic acid; 2 - hydroxy - 3 - methoxy - benzoic acid; 1 - hydroxy - 2 - naphthoic acid; salicyluric acid; or the sodium salts thereof.

7. The process according to any one of Claims 1 to 6 wherein soft gelatin capsule is prepared containing said drug and said adjuvant.

**Revendications pour les Etats Contractants: BE, CH, DE, FR, GB, IT, LI, LU, NL, SE**

1. Un suppositoire pour l'administration par voie rectale comprenant une quantité thérapeutique efficace d'un médicament pouvant être absorbé dans la circulation sanguine à partir d'un compartiment rectal et un adjuvant de formule:

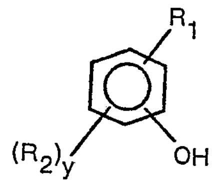

dans laquelle $R_1$ est $CO_2H$, $(CH_2)COOH$,

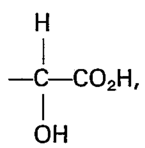

ou un sel acceptable en pharmacie correspondant, où $R_2$ est OH, H, un alcoxy de 1 à 10 atomes de carbone, un alkyle de 1 à 10 atomes de carbone, un radical halogéno ou un radical trihalogénoalkyle de 1 à 5 atomes de carbone, et ù y est 1 ou 2, à l'exception des suppositoires rectaux contenant une combinaison d'indométhacine, d'acide niflumique, de phényl-butazone ou d'acide acétylsalicylique conjointement à de l'acide salycilique ou un salicylate de métal alcalin.

2. Suppositoire selon la revendication 1, dans lequel ledit médicament comprend les xanthines, des agents anti-cancéreux, des antibiotiques, des polypeptides, des anti-arythmiques, d'autres agents cardiovasculaires, des anti-diabétiques, des antiulcéreux, des antifongiques, des anti-nauséeux, des sédatifs, des diurétiques, des antihypertenseurs et des médicaments pour le traitement de la maladie de Parkinson.

3. Suppositoire selon la revendication 1, dans lequel ledit médicament comprend le chloramphénicol, la pénicilline ou la céphalosporine.

4. Suppositoire selon la revendication 1, dans lequel ledit médicament comprend la quinidine, la lidocaïne, la théophylline, la L-dopa, le céfmétazole, l'insuline, la céfoxitine, l'héparine, le rouge de phénol, la pénicilline G ou la gentamycine.

5. Suppositoire selon la revendication 1, dans lequel ledit médicament comprend l'amiloride, l'hydrochlorothiazide, le moduretic, la cabidopa, la lévodopa, la céfoxitine, le céfmétazole, la cyclobenzaprine, le diflunisal, l'indométhacine, la méthyldopa, le sulindac, le timolol, le chlorhydate de (−) - 1 - (cyclopropylméthyl) - 4 - [3 - (trifluorométhylthio) - 5H - dibenzo[a,d]cycloheptène - 5 - ylidène]pipéridine, le maléate de N - [(S) - 1 - (éthoxycarbonyl) - 3 - phénylpropyl] - L - alanyl - L - proline, l'oxalate de (+) - 10,11 - dihydro - 5 - méthyl - 5H - dibenzo[a,d]cycloheptène - 5,10 - imine, l'acide 1 - éthyl - 6 - fluoro - 1,4 - hihydro - 4 - oxo - 7 - (1 - pipérazinyl) - 3 - quinoléine - carboxylique, la 3 - fluoro - D - alanine et le sel de sodium hémihydraté de la D - 4 - (1 - méthyl - 3 - oxo - 1 - buténylamino) - 3 - isoxazolidinone, le L - N - (2 - oxopipéradine - 6 - ylcarbonyl) - histidyl - L - thiazolidine - 4 - carboxamide, la N - formimidoyl thiénamycine monohydratée, l'acide (6,7 - dichloro - 2 - méthyl - 2 - phényl - 1 - oxo - 5 - indanyloxy)acétique, l'ibuprofène, le naproxen, l'acide 5 - (4 - chlorobenzoyl) - 1,4 - diméthyl - 1H - pyrrole - 2 - acétique, la phénylbutazone, la dexaméthasone, la prednisolone, la clonidine, le propranolol, le diazépam, le chlordiazépoxyde, le furosémide, le céfamandole, l'acide nalidixique, l'amoxicilline, l'halopéridol et le captopril.

6. Suppositoire selon l'une quelconque des revendications 1 à 5, dans lequel ledit adjuvant est l'acide 5 - méthoxysalicylique, l'acide salicylique, l'acide homovanillique, l'acide 2,5 - dihydroxybenzoïque,

l'acide 2,4 - dihydroxybenzoïque, l'acide 3,4 - dihydroxymandélique, l'acide 3 - méthoxy - 4 - hydroxy - cinnamique, l'acide 5 - méthoxy - 2 - hydroxy - phénylsulfonique, l'acide 3 - méthylsalicylique, l'acide 5 - méthylsalicylique, l'acide 5 - tert - octylsalicylique, l'acide 3 - tert - butyl - 6 - méthylsalicylique, l'acide 3,5 - di - isopropylsalicylique, l'acide 3 - tert - butyl - 5 - méthylsalicylique, l'acide gaïacolsulfonique, l'acide 5 - bromosalicylique, l'acide 3,5 - dibromosalicylique, l'acide 5 - iodosalicylique, l'acide 3,5 - diiodosalicylique, l'acide 2 - hydroxyphénylacétique, l'acide 3 - hydroxy - 2 - naphtoïque, l'acide mandélique, l'acide phényllactique, l'acide 2 - hydroxyphénylméthanesulfonique, l'acide 5 - trifluorométhyl - 2 - hydroxybenzoïque, l'acide 4 - hydroxy - 3 - hydroxyphénylméthanesulfonique, l'acide 3 - méthoxy - salicylique, l'acide 5 - octyloxysalicylique, l'acide 5 - butoxy - salicylique, l'acide p - hydroxyphénoxyacétique, l'acide 3,4 - dihydroxyphénylacétique, l'acide 5 - chlorosalicylique, l'acide 3,4 - dihydroxycinnamique, l'acide 3,5 - dihydroxybenzoïque, l'acide 2 - hydroxy - 3 - méthoxybenzoïque, l'acide 1 - hydroxy - 2 - naptoïque, l'acide salicylurique, ou leurs sels de sodium.

7. Suppositoire selon l'une quelconque des revendications 1 à 6, comprenant une capsule de gélatine élastique souple contenant ledit médicament et ledit adjuvant.

**Revendications pour l'Etat Contractant: AT**

1. Un procédé pour préparer un suppositoire pour l'administration par voie rectale par combinaison de façon connue en soi d'une quantité thérapeutique efficace d'un médicament pouvant être absorbé dans la circulation sanguine à partir d'un compartiment rectal et d'un adjuvant de formule:

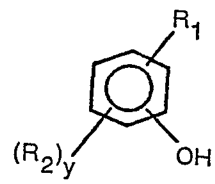

dans laquelle $R_1$ est $CO_2H$, $(CH_2)COOH$,

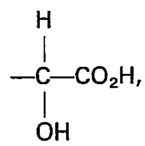

ou un sel acceptable en pharmacie correspondant, où $R_2$ est OH, H, un alcoxy de 1 à 10 atomes de carbone, un alkyle de 1 à 10 atomes de carbone, un radical halogéno ou un radical trihalogénoalkyle de 1 à 5 atomes de carbone, et où y est 1 ou 2, à l'exception des suppositoires rectaux contenant une combinaison d'indométhacine, d'acide niflumique, de phénylbutazone ou d'acide acétylsalicylique conjointement à de l'acide salicylique ou un salicylate de métal alcalin.

2. Le procédé selon la revendication 1, dans lequel ledit médicament comprend des xanthines, des agents anticancéreux, des antibiotiques, des polypeptides, des antiarythmiques, d'autres agents cardiovasculaires, des antidiabétiques, des antiulcéreux, des antifongiques, des antinauséeux, des sédatifs, des diurétiques, des antihypertenseurs et des médicaments pour le traitement de la maladie de Parkinson.

3. Le procédé selon la revendication 1, dans lequel ledit médicament comprend le chloramphénicol, la pénicilline ou la céphalosporine.

4. Le procédé selon la revendication 1, dans lequel ledit médicament comprend la quinidine, la lidocaïne, la théophylline, la L-dopa, le céfmétazole l'insuline, la céfoxitine, l'héparine, le rouge de phénol, la pénicilline G ou la gentamycine.

5. Le procédé selon la revendication 1, dans lequel ledit médicament comprend l'amiloride, l'hydrochlorothiazide, le modurétic, la carbidopa, la lévodopa, la céfoxitine, le céfmétazole, la cyclobenzaprine, le diflunisal, l'indométhacine, la méthyldopa, le sulindac, le timolol, le chlorhydrate de (−) - 1 - (cyclopropylméthyl) - 4 - [3 - (trifluorométhylthio) - 5H - dibenzo[a,d]cycloheptène - 5 - ylidène]pipéridine, le maléate de N - [(S) - 1 - (éthoxycarbonyl) - 3 - phénylpropyl] - L - alanyl - L - proline, l'oxalate de (+) - 10,11 - dihydro - 5 - méthyl - 5H - dibenzo[a,d]cycloheptène - 5,10 - imine, l'acide 1 - éthyl - 6 - fluoro - 1,4 - dihydro - 4 - oxo - 7 - (1 - pipérazinyl) - 3 - quinoléine - carboxylique, la 3 - fluoro - D - alanine et le sel de sodium hémihydraté de la D - 4 - (1 - méthyl - 3 - oxo - 1 - buténylamino) - 3 - isoxazolidinone, le L - N - (2 - oxopipéridine - 6 - ylcarbonyl) - histidyl - L - thiazolidine - 4 - carboxamide, la N - formimidoyl thiénamycine monohydratée, l'acide (6,7 - dichloro - 2 - méthyl - 2 - phényl - 1 - oxo - 5 - indanyloxy)acétique, l'ibuprofène, le naproxen, l'acide 5 - (4 - chlorobenzyl) - 1,4 - diméthyl - 1H - pyrrole - 2 - acétique, la phénylbutazone, la dexméthasone, la prédnisolone, la clonidine, le propranolol, le diazépam, le chlordiazépoxyde, le furosémide, le céfamandole, l'acide nalidixique, l'amoxicilline, l'halopéridol et le captopril.

6. Suppositoire selon l'une quelconque des revendications 1 à 5, dans lequel ledit adjuvant est l'acide

5 - méthoxysalicylique, l'acide salicylique, l'acide homovanillique, l'acide 2,5 - dihydroxybenzoïque, l'acide 2,4 - dihydroxybenzoïque, l'acide 3,4 - dihydroxymandélique, l'acide 3 - méthoxy - 4 - hydroxy - cinnamique, l'acide 5 - méthoxy - 2 - hydroxyphénylsulfonique, l'acide 3 - méthylsalicylique, l'acide 5 - méthylsalicylique, l'acide 5 - tert - octylsalicylique, l'acide 3 - tert - butyl - 6 - méthylsalicylique, l'acide 3,5 - diisopropylsalicylique, l'acide 3 - tert - butyl - 5 - méthylsalicylique, l'acide gaïacolsulfonique, l'acide 5 - bromosalicylique, l'acide 3,5 - dibromosalicylique, l'acide 5 - iodosalicylique, l'acide 3,5 - diiodosalicylique, l'acide 2 - hydroxyphénylacétique, l'acide 3 - hydroxy - 2 - naphtoïque, l'acide mandélique, l'acide phényllactique, l'acide 2 - hydroxyphénylméthanesulfonique, l'acide 5 - trifluorométhyl - 2 - hydroxybenzoïque, l'acide 4 - hydroxy - 3 - hydroxyphénylméthanesulfonique, l'acide 3 - méthoxy - salicylique, l'acide 5 - octyloxysalicylique, l'acide 5 - butoxy - salicylique, l'acide p - hydroxyphénoxy - acétique, l'acide 3,4 - dihydroxyphénylacétique, l'acide 5 - chlorosalicylique, l'acide 3,4 - dihydroxycinnamique, l'acide 3,5 - dihydroxybenzoïque, l'acide 2 - hydroxy - 3 - méthoxy - benzoïque, l'acide 1 - hydroxy - 2 - naphtoïque, l'acide salicylique, ou leurs sels de sodium.

7. Le procédé selon l'une quelconque des revendications 1 à 6, dans lequel on prépare une capsule de gélatine élastique souple contenant ledit médicament et ledit adjuvant.

**Patentansprüche für die Vertragsstaaten: BE, CH, DE, FR, GB, IT, LI, LU, NL, SE**

1. Ein rektal verabreichbares Zäpfchen, enthaltend eine therapeutisch wirksame Menge eines Arzneimittels, das zur Absorption aus einem Rektalsektor in den Blutstrom geeignet ist, und einen Hilfsstoff der Formel

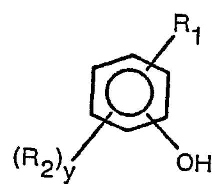

worin $R_1$ $CO_2H$, $(CH_2)COOH$,

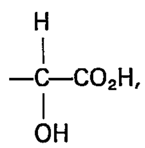

$SO_3H$ oder ein pharmazeutisch annehmbares Salz davon ist, worin $R_2$ OH, H, Alkoxy mit 1—10 Kohlenstoffatomen, Alkyl mit 1—10 Kohlenstoffatomen, ein Halogenrest oder ein Trihalogenalkyl mit 1—5 Kohlenstoffatomen ist, und worin y eine ganze Zahl von 1 oder 2 ist, mit der Ausnahme von Rektalzäpfchen, die eine Kombination von Indomethacin, Nifluminsäure, Phenylbutazon oder Acetylsalicylsäure zusammen mit Salicylsäure oder einem Alkalimetallsalicylat enthalten.

2. Zäpfchen des Anspruchs 1, worin das genannte Arzneimittel Xanthine, Antikrebsmittel, Antibiotika, Polypeptide, Antiarrhythmika, andere kardiovaskuläre Mittel, Antidiabetika, Antiulcusmittel, Antifungusmittel, Antinauseata, Sedativa, Diuretika, antihypertensive Mittel und Arzneimittel zur Behandlung der Parkinson'schen Krankheit umfaßt.

3. Zäpfchen des Anspruchs 1, worin das genannte Arzneimittel Chloramphenicol, Penicillin oder Cephalosporin umfaßt.

4. Zäpfchen des Anspruchs 1, worin das genannte Arzneimittel Chinidin, Lidocain, Theophyllin, L-Dopa, Cefmetazol, Insulin, Cefoxitin, Heparin, Phenolrot, Penicillin G oder Gentamycin umfaßt.

5. Zäpfchen des Anspruchs 1, worin das genannte Arzneimittel Amilorid, Hydrochlorothiazid, Moduretic, Carbidopa, Levodopa, Cefoxitin, Cefmetazol, Cyclobenzaprin, Diflunisal, Indomethacin, Methyldopa, Sulindac, Timolol, (−) - 1 - (Cyclopropylmethyl) - 4 - [3 - (trifluoromethylthio) - 5H - dibenzo(a,d)cyclohepten - 5 - yliden] - piperidinhydrochlorid, N - [(S) - 1 - (Ethoxycarbonyl) - 3 - phenylpropyl] - L - alanyl - L - prolinmaleat, (+)10,11 - Dihydro - 5 - methyl - 5H - dibenzo[a,d]cyclohepten - 5,10 - iminoxalat, 1 - Ethyl - 6 - fluor - 1,4 - dihydro - 4 - oxo - 7 - (1 - piperazinyl) - 3 - chinolin - carbonsäure, 3 - Fluor - D - alanin und D - 4 - (1 - Methyl - 3 - oxo - 1 - butenylamino) - 3 - isoxazolidinon - Natriumsalz - hemihydrat, L - N - (2 - Oxopiperidin - 6 - ylcarbonyl) - histidyl - L - thiazolidin - 4 - carboxamid, N - Formimidoylthienamycinmonohydrat, (6,7 - Di - chlor - 2 - methyl - 2 - phenyl - 1 - oxo - 5 - indanyloxy)essigsäure, Ibuprofen, Naproxen, 5 - (4 - Chlorbenzoyl) - 1,4 - dimethyl - 1H - pyrrol - 2 - essigsäure, Phenylbutazon, Dexamethason, Prednisolon, Clonidin, Propranolol, Diazepam, Chlorodiazeproxid, Furosemid, Cephamandol, Nalidixinsäure, Amoxicillin, Haloperidol und Captopril umfaßt.

6. Zäpfchen nach einem der Ansprüche 1 bis 5, worin der genannte Hilfsstoff 5 - Methoxysalicylsäure, Salicylsäure, Homovanillinsäure, 2,5 - Dihydroxybenzoesäure; 2,4 - Dihydroxybenzoesäure; 3,4 - Dihydroxymandelsäure; 3 - Methoxy - 4 - hydroxyzimtsäure; 5 - Methoxy - 2 - hydroxyphenylsulfonsäure; 3 - Methylsalicylsäure; 5 - Methylsalicylsäure; 5 - tert.Octylsalicylsäure; 3 - tert.Butyl - 6 - methylsalicylsäure; 3,5 - Diisopropylsalicylsäure; 3 - tert.Butyl - 5 - methylsalicylsäure; Guaikolsulfonsäure; 5 - Bromsalicylsäure; 3,5 - Dibromsalicylsäure, 5 - Iodsalicylsäure; 3,5 - Diiodsalicylsäure; 2 - Hydroxyphenylessigsäure; 3 - Hydroxy - 2 - naphthoesäure; Mandelsäure; Phenylmilchsäure; 2 - Hydroxyphenylmethansulfonsäure; 5 - Trifluor - methyl - 2 - hydroxybenzoesäure; 4 - Hydroxy - 3 - hydroxyphenylmethansulfonsäure, 3 - Methoxysalicylsäure; 5 - Octyloxysalicylsäure; 5 - Butoxysalicylsäure; p - Hydroxyphenoxyessigsäure, 3,4 - Dihydroxyphenylessigsäure; 5 - Chlorsalicylsäure; 3,4 - Dihydroxyzimtsäure; 3,5 - Dihydroxybenzoesäure; 2 - Hydroxy - 3 - methoxybenzoesäure; 1 - Hydroxy - 2 - naphthoesäure; Salicylharnsäure; oder die Natriumsalze davon ist.

7. Zäpfchen nach einem der Ansprüche 1 bis 6, umfassend eine weiche, elastische Gelatinekapsel, die das genannte Arzneimittel und den genannten Hilfsstoff enthält.

**Patentansprüche für den Vertragsstaat: AT**

1. Ein Verfahren zur Herstellung eines rektal verabreichbaren Zäpfchens, bei dem in an sich bekannter Weise eine therapeutisch wirksame Menge eines Arzneimittels, das zur Absorption aus dem Rektalsektor in den Blutstrom geeignet ist, und ein Hilfsstoff der Formel:

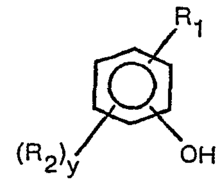

worin $R_1$ $CO_2H$, $(CH_2)COOH$,

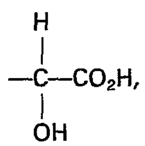

$SO_3H$ oder ein pharmazeutisch annehmbares Salz davon ist, worin $R_2$ OH, H, Alkoxy mit 1—10 Kohlenstoffatomen, Niedrigalkyl mit 1—10 Kohlenstoffatomen, ein Halogenrest oder ein Trihalogenalkyl mit 1—5 Kohlenstoffatomen ist, und worin y eine ganze Zahl von 1 oder 2 ist, mit der Ausnahme einer Kombination von Indomethacin, Nifluminsäure, Phenylbutazon oder Acetylsalicylsäure zusammen mit Salicylsäure oder einem Alkalimetallsalicylat, formuliert werden.

2. Das Verfahren des Anspruchs 1, worin das genannte Arzneimittel Xanthine, Antikrebsmittel, Antibiotika, Polypeptide, Antiarrhythmika, andere kardiovaskuläre Mittel, Antidiabetika, Antiulcusmittel, Antifungusmittel, Antinauseata, Sedativa, Diuretika, antihypertensive Mittel und Arzneimittel zur Behandlung der Parkinson'schen Krankheit umfaßt.

3. Das Verfahren des Anspruchs 1, worin das genannte Arzneimittel Chloramphenicol, Penicillin oder Cephalosporin umfaßt.

4. Das Verfahren des Anspruchs 1, worin das genannte Arzneimittel Chinidin, Lidocain, Theophyllin, L-Dopa, Cefmetazol, Insulin, Cefoxitin, Heparin, Phenolrot, Penicillin G oder Gentamycin umfaßt.

5. Das Verfahren des Anspruchs 1, worin das genannte Arzneimittel Amilorid, Hydrochlorothiazid, Moduretic, Carbidopa, Levodopa, Cefoxitin, Cefmetazol, Cyclobenzaprin, Diflunisal, Indomethacin, Methyldopa, Sulindac, Timolol, (—) - 1 - (Cyclopropylmethyl) - 4 - [3 - (trifluormethylthio) - 5H - dibenzo(a,d)cyclohepten - 5 - yliden] - piperidinhydrochlorid, N - [(S) - 1 - (Ethoxycarbonyl) - 3 - phenylpropyl] - L - alanyl - L - prolinmaleat, (+)10,11 - Dihydro - 5 - methyl - 5H - dibenzo[a,d]cyclohepten - 5,10 - iminoxalat, 1 - Ethyl - 6 - fluor - 1,4 - dihydro - 4 - oxo - 7 - (1 - piperazinyl) - 3 - chinolin - carbonsäure, 3 - Fluor - D - alanin und D - 4 - (1 - Methyl - 3 - oxo - 1 - butenylamino) - 3 - isoxazolidinon - Natriumsalz - hemihydrat, L - N - (2 - Oxopiperidin - 6 - ylcarbonyl) - histidyl - L - thiazolidin - 4 - carboxamid, N - Formimidoylthienamycinmonohydrat, (6,7 - Dichlor - 2 - methyl - 2 - phenyl - 1 - oxo - 5 - indanyloxy)essigsäure, Ibuprofen, Naproxen, 5 - (4 - Chlorbenzoyl) - 1,4 - dimethyl - 1H - pyrrol - 2 - essigsäure, Phenylbutazon, Dexamethason, Prednisolon, Clonidin, Propranolol, Diazepam, Chlorodiazeproxid, Furosemid, Cephamandol, Nalidixinsäure, Amoxicillin, Haloperidol und Captopril umfaßt.

6. Das Verfahren nach einem der Ansprüche 1 bis 5, worin der genannte Hilfsstoff 5 - Methoxysalicylsäure, Salicylsäure, Homovanillinsäure; 2,5 - Dihydroxybenzoesäure; 2,4 - Dihydroxybenzoesäure; 3,4 - Dihydroxymandelsäure; 3 - Methoxy - 4 - hydroxyzimtsäure; 5 - Methoxy - 2 - hydroxyphenylsulfonsäure; 3 - Methylsalicylsäure; 5 - Methylsalicylsäure; 5 - tert.Octylsalicylsäure; 3 - tert.Butyl - 6 - methylsalicylsäure; 3,5 - Diisopropylsalicylsäure; 3 - tert.Butyl - 5 - methylsalicylsäure; Guaikolsulfonsäure; 5 - Bromsalicylsäure; 3,5 - Dibromsalicylsäure; 5 - Iodsalicylsäure; 3,5 - Diiodsalicylsäure; 2 - Hydroxyphenylessigsäure; 3 - Hydroxy - 2 - naphthoesäure; Mandelsäure;

Phenylmilchsäure; 2 - Hydroxyphenylmethansulfonsäure; 5 - Trifluor - methyl - 2 - hydroxybenzoesäure; 4 - Hydroxy - 3 - hydroxyphenylmethanesulfonsäure; 3 - Methoxysalicylsäure; 5 - Octyloxysalicylsäure; 5 - Butoxysalicylsäure; p - Hydroxyphenoxyessigsäure; 3,4 - Dihydroxyphenylessigsäure; 5 - Chlorsalicylsäure; 3,4 - Dihydroxyzimtsäure; 3,5 - Dihydroxybenzoesäure; 2 - Hydroxy - 3 - methoxybenzoesäure; 1 - Hydroxy - 2 - naphthoesäure; Salicylharnsäure; oder die Natriumsalze davon ist.

7. Das Verfahren nach einem der Ansprüche 1 bis 6, worin eine Weichgelatinekapsel hergestellt wird, die das genannte Arzneimittel und den genannten Hilfsstoff enthält.

Fig. 1

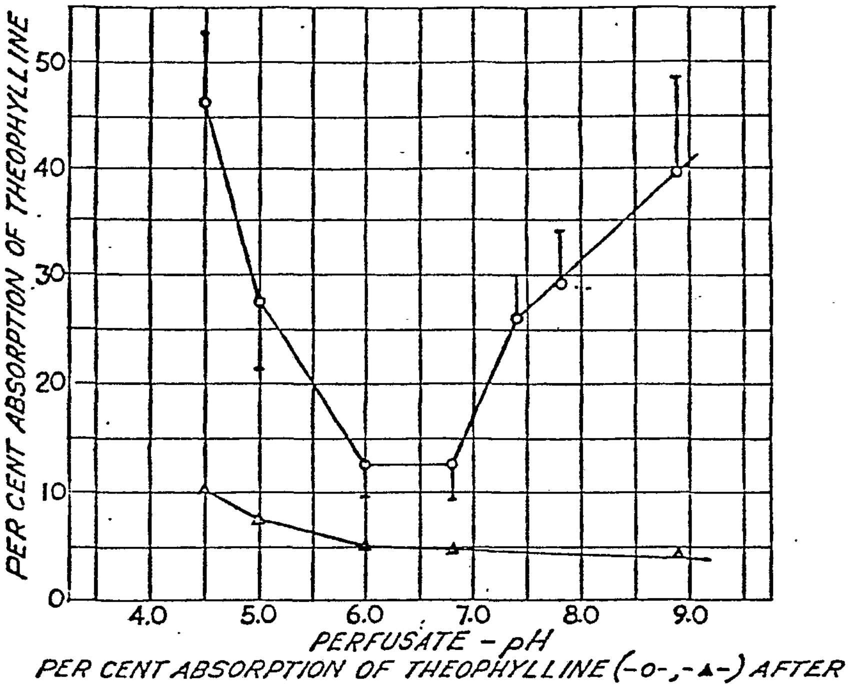


PER CENT ABSORPTION OF THEOPHYLLINE (-o-, -▲-) AFTER 1 HR FROM PERFUSATE IN THE RAT RECTUM UNDER SAME CONDITIONS. -o-; INITIAL CONCENTRATION = 0.2 % SODIUM SALICYLATE AND 200 MG/ML THEOPHYLLINE, -▲-; INITIAL CONCENTRATION = 200 MG/ML THEOPHYLLINE, ONLY.

Fig. 3

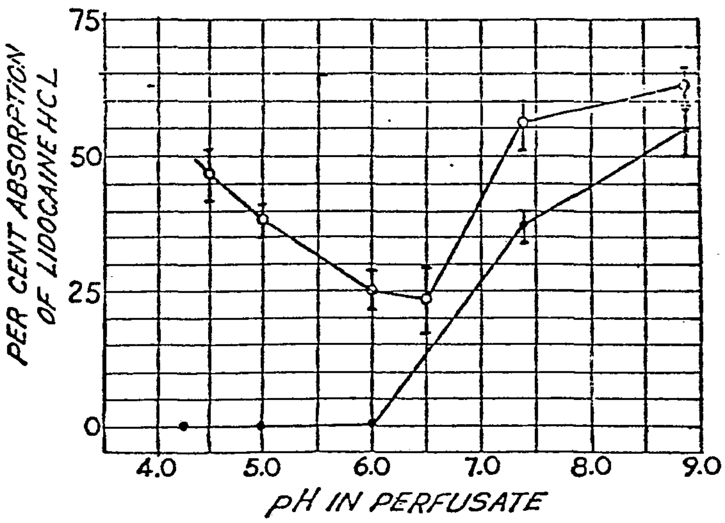


18. EFFECT OF pH AND SALICYLATE ON THE DISAPPEARANCE OF LIDOCAINE HCL FROM A. PERFUSATE IN THE RAT RECTUM o = 0.5 % SODIUM SALICYLATE, • = NO SODIUM SALICYLATE. INITIAL LIDOCAINE HCL CONCENTRATION WAS 500 MG/ML

Fig.2

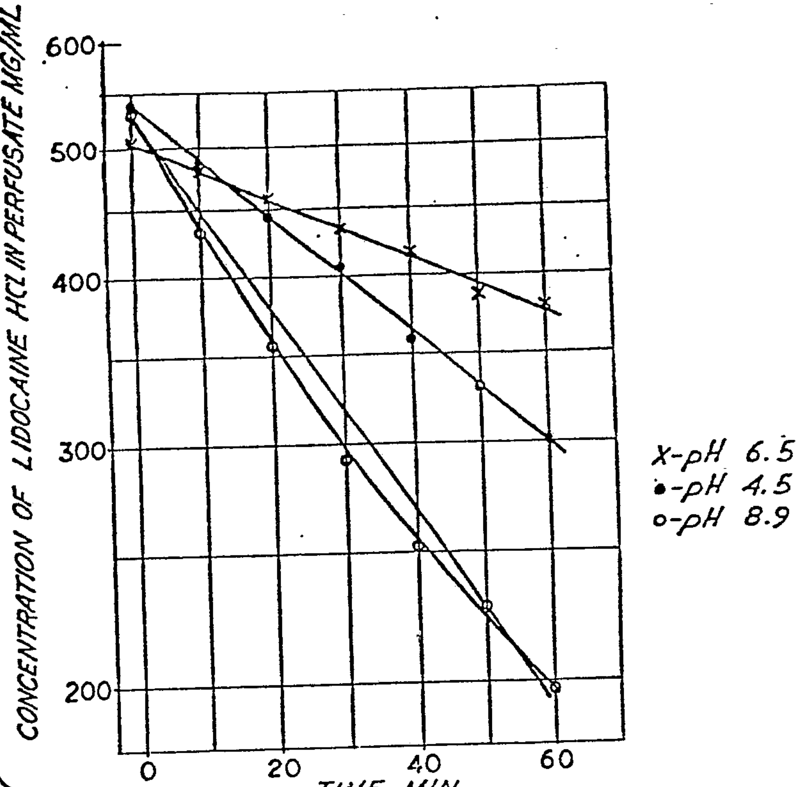


EFFECT OF pH ON THE DISAPPEARANCE OF LIDOCAINE FROM THE RAT RECTUM IN THE PRESENCE OF 0.5 % SODIUM SALICYLATE

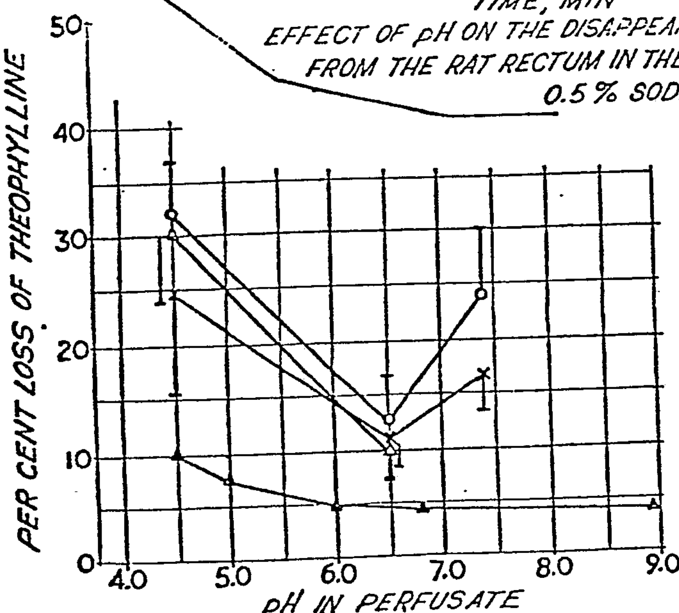


Fig.4

EFFECT OF pH AND DIHYDROXY BENZOATE ON THE DISAPPEARANCE OF THEOPHYLLINE FROM PERFUSATE IN THE RAT RECTUM AFTER 1 HR. o = 0.5 % SODIUM 2,4-DIHYDROXY BENZOATE, △ = 0.5 % SODIUM 2,5 DIHYDROXY-BENZOATE, X = 0.5/3.5 DIHYDROXY BENZOATE, ▲ = NO ADDITIVE